# EUROPEAN PATENT APPLICATION

(11) **EP 4 344 601 A1**
(43) Date of publication of application: **03.04.2024**
(21) Application number: 22198240.8
(22) Date of filing: 28.09.2022
(51) Int. Cl.: A61B 1/00, A61B 1/018

(54) **ENDOSCOPE COMPRISING A FRICTION-REDUCING WORKING CHANNEL TUBE**

(71) Applicant: Ambu A/S, 2750 Ballerup (DK)
(72) Inventor: MATTHISON-HANSEN, Kaspar Mat, 3000 Helsingør (DK)
(74) Representative: Winter, Brandl - Partnerschaft mbB

(57) **Abstract**

The present disclosure relates to an endoscope (2) comprising a proximal endoscope handle (4) or interface comprising a handle or interface housing and a working channel access port (16), an insertion cord (6) extending distally from the endoscope handle (4) or interface, configured to be inserted into a patient's body cavity and comprising at least an insertion tube (8) and a distal tip unit (12) and a working channel (14) extending from the working channel access port (16) of the endoscope handle (4) or interface to the distal tip unit (12) of the insertion cord (6). The working channel (14) comprises a working channel tube (18) arranged at least in sections inside the insertion cord (6) wherein the working channel tube (18) comprises an inner friction-reducing textured or structured surface (24) and an outer surface (26). The present disclosure further relates to a method of producing a working channel tube (18).

## Description

The present disclosure relates to an endoscope comprising a working channel tube and a method for producing a working channel tube of an endoscope.

### Related art

Known endoscopes usually comprise: an endoscope handle or interface comprising a handle or interface housing and a working channel access port; an insertion cord configured to be inserted into a patient's body cavity and comprising at least an insertion tube and a distal tip unit; and an internal working channel extending from the working channel access port of the endoscope handle or interface to the distal tip unit of the insertion cord. Optionally, the insertion cord may comprise an actively bendable bending section in addition to the insertion tube and the distal tip unit.

Working channels are usually formed by a connector part, the so-called biopsy connector or Y-connector, a flexible working channel tube arranged inside the endoscope handle or interface, the insertion tube and the bending section, and a tip housing of the distal tip unit. A surgical instrument may be guided through the working channel into the patient's body cavity, i.e. distally with respect to the tip of the endoscope. Additionally, the working channel is usually also used as a suction channel to aspirate for example body fluid from an operation area within the patient's body cavity.

The flexible working channel tube of the working channel is usually formed as an elongated hollow tube body, usually made of a plastic material.

The working channel often has a small cross-section in relation to its length. Particularly in the case of long working channels or in bends of the working channel, in particular of the working channel tube, a tool inserted by a surgeon may be subjected to increased frictional resistance, which worsens handling for the surgeon and thus patient safety.

Therefore, it is basically desirable to reduce friction in the working channel. It is in this light already known to provide coated working channel tubes, for example with Teflon, to reduce friction in the working channel tube. Coating the working channel tube is complex and expensive, particularly regarding endoscopes comprising a long working channel, so that it is not a practical solution, especially for single use endoscopes.

### Brief description of the disclosure

It is therefore an object of the present disclosure to provide an endoscope that eliminates or at least reduces the disadvantages of the prior art. Specifically, it is the object of the present disclosure to provide an endoscope having a working channel which has low friction in the working channel while being cost-effective to manufacture.

This object is solved by an endoscope according to claim 1, by a system according to claim 9 and by a method for producing a working channel tube of an endoscope in accordance with claim 10. Advantageous aspects of the present disclosure are claimed in the dependent claims and/or are described herein below.

The present disclosure relates to an endoscope comprising: a proximal endoscope handle or interface comprising a handle or interface housing and a working channel access port; an insertion cord extending distally from the endoscope handle or interface, configured to be inserted into a patient's body cavity and comprising at least an insertion tube and a distal tip unit; and a working channel extending from the working channel access port of the endoscope handle or interface to the distal tip unit of the insertion cord and comprising a working channel tube arranged at least in sections inside the insertion cord, the working channel tube comprising an inner, i.e. inside friction-reducing textured or structured surface and an outer, i.e. outside surface.

It is to be understood that a working channel tube is a tubular, substantially thin-walled element configured to allow a practitioner to insert medical tools and equipment into the patient's body cavity through the endoscope and/or to expel patient material from the patient through the endoscope. The working channel tube may preferably have a circular cylindrical shape with a constant cross-section over a longitudinal extension of the working channel tube. However, embodiments are also conceivable which have an oval-cylindrical shape or a polygonal-cylindrical shape. Furthermore, it is conceivable that the cross-section of the working channel tube tapers or widens over the longitudinal extension.

Moreover, it is to be understood, that the working channel access port is an opening that allows the practitioner to insert the medical tools and equipment into the working channel. The working channel access port may preferably be designed as a Y-connector.

Further, it is to be understood, that "arranged at least in sections inside the insertion cord" means that at least a defined section of the working channel tube in an axial extension of the working channel tube is arranged in the insertion cord in such a way, that the working channel tube in this defined section is completely surrounded by the insertion cord in a radial direction.

In addition, it is to be understood, that "comprising an inner friction-reducing textured or structured surface" means that at least a part or portion in a longitudinal direction of the working channel inside surface is textured or structured.

Furthermore, it is to be understood, that a "textured or structured surface" is a surface, which, after the tube body has been initially shaped, has been modified in such a way as to increase the structuring or texturing of the surface, for example by machining or chemical treatment.

It is to be understood, that structure or texture is an ordered or disordered unevenness of the surface. Specifically, the surface is provided with geometrically ordered or disordered indentations and/or protrusions, where a dimension of the indentations and/or protrusions is, preferably much, smaller than a dimension of the object, namely the working channel tube, formed with the surface.

Accordingly, the core of the present disclosure is, that the endoscope according to the present disclosure comprises the working channel tube arranged at least in sections in, i.e. inside, the insertion cord, wherein the inner surface of the working channel tube has a surface relief with protrusions and/or indentations.

Such a surface of the working channel tube may reduce a contact area between the medical tool or device inserted into the working channel by the practitioner and the working channel tube itself. This may significantly reduce frictional resistance between the working channel tube and the medical tool or device, which must be overcome by the practitioner when inserting the tool or device into the working channel. This allows the practitioner to make finer and more precise movements, and increases treatment comfort for the practitioner and a patient safety. This may particularly be the case when the insertion cord and thereby the working channel tube is in a curved and/or bent or partly bent state where a tool may be more likely to contact the inner surface of the working channel during insertion or extraction of the tool.

Preferably, the inner friction-reducing textured or structured surface may be a roughened surface.

In other words, the textured or structured surface may be a surface that has been roughened by mechanical, chemical or thermal treatment after the macroscopic geometry of the working channel tube has been manufactured.

Mechanical roughening may be understood as abrasive roughening, for example by means of grinding, embossing roughening with an embossing tool, sand or glass bead blasting or the like.

Chemical roughening may be understood as surface etching or the like.

Thermal roughening may be understood as a heat treatment in which, for example, particles or the like are detached from the surface by a heat treatment leaving indentations.

Alternatively, it may be conceivable to introduce particles into the main material of the working channel tube during the primary forming stage, which leads to a roughened surface, or to form the main material during the primary forming stage in such a way that the surface is a roughened surface e.g. by a corresponding mold or core.

Preferably, the inner friction-reducing textured or structured surface may be more structured or textured, preferably may be rougher than the outer surface.

In other words, a roughness of the inner surface of the working channel tube may be greater than a roughness of the outer surface of the working channel tube. The outer surface may be unprocessed or unmachined. This means that the outer surface may be the surface that is created in the initially forming process of the working channel tube, for example by extrusion.

Alternatively, the outer surface may be textured or structured, in particular roughened. The outer surface may be formed by the same or equivalent methods as the inner surface. In particular, depending on the method or treatment applied for texturing or structuring, in particular roughening, the inner surface, it may be easier to treat both the inner surface and the outer surface, i.e. not only the inner surface. This alternative embodiment may thus be advantageous in a manufacturing point of view.

Roughness is understood to be a measurable quantity, for example Ra or Rz. Ra is the more common measured quantity. Ra is the arithmetic mean roughness value according to DIN EN ISO 4287:2010. To determine this measured value, the surface is scanned on a defined measuring section and all height and depth differences of the surface are recorded. After calculating the determined integral of this roughness curve on the measuring section, this result is finally divided by the length of the measuring section. The range of roughness values extends from 25 µm for very rough surfaces with noticeable scoring to less than 0.1 µm with machining marks that are no longer visible.

It is also conceivable that the textured or structured surface may be obtained by applying pressure, in particular high pressure, to the inner surface of the working channel tube, in order to change the material properties of the material of the working channel tube, so as to reduce friction.

Preferably, the working channel tube may comprise an inner layer or tube having the inner friction-reducing textured or structured surface and an outer layer or tube having the outer surface.

In other words the working channel tube may have a layered structure. This means that the working channel tube may have several successive layers in a radial direction of the working channel tube. In particular, it may be exactly two layers. The different layers may be formed directly adjacent to each other and be connected to each other in a form-fit and/or force-fit manner and/or adhesively, i.e. via adhesive bond. The different layers may, for example, be glued or welded to each other and/or formed together as a single piece. A thickness of the different layers in radial direction may be different. Alternatively, however, it is also conceivable that the layers have the same thickness in the radial direction. The different layers, namely the inner layer and the outer layer, may be made of different materials with different material properties. For example, the outer layer may be made of a soft, kink-resistant material and the inner layer may be made of a material with good sliding properties. Of course, other combinations of material properties are also conceivable.

Both the inner layer and the outer layer may preferably be made of a thermoplastic or thermosetting material. The outer layer may e.g. be made of TPU (thermoplastic polyurethane) and the inner layer may be made of polyethylene, preferably HDPE (high-density polyethylene). For the inner layer, also PTFE (polytetrafluoroethylene) may be a suitable thermoplastic material. Thermosetting polymers such as fluoropolymers may also be suitable.

Preferably, the melting temperatures of the different layers may differ. In this way, manufacturing may be simplified and, in particular, the forming of the inner surface (structure or texture) may be simplified.

The inner layer may be thinner than the outer layer, or said differently, a wall thickness of the inner layer may be smaller than a wall thickness of the outer layer. E.g. the inner layer may have a wall thickness of between 0.01 mm to 0.1 mm, especially preferred of between 0.050 mm to 0.080 mm, and the outer layer may have a wall thickness of between 0.1 mm to 1 mm, especially preferred of between 0.32 mm to 0.35 mm. However, other wall thicknesses are conceivable and it is also conceivable that the wall thickness of the inner layer is greater than the wall thickness of the outer layer.

Alternatively, more than two layers are possible. For example, a bridge layer may be formed between the outer layer and the inner layer. This bridge layer may be applied, for example, to prevent material incompatibilities between the outer layer and the inner layer. This is e.g. of advantage if the outer layer is made of TPU (thermoplastic polyurethane) and the inner layer is made of HDPE (high-density polyethylene). The bridge layer may also be applied, for example, to improve the bending and kinking properties of the working channel tube.

Preferably, the inner friction-reducing textured or structured surface may have the arithmetic mean roughness value Ra according to DIN EN ISO 4287:2010 and the arithmetic mean roughness value Ra is greater than or equal to 1.0 µm, preferably greater than or equal to 1.5 µm.

In other words, the average roughness Ra of the inner surface of the working channel tube may be greater than or equal to 1.0 µm, preferably greater than or equal to 1.5 µm.

In still other words, the average roughness Ra of the inner surface of the working channel tube may be such that a contact area between the medical tool or the like in the working channel and the working channel tube is reduced, preferably by at least 20 %, especially preferred by at least 40 %, compared to a scenario where the inner surface corresponds to the unprocessed surface after extrusion.

Preferably, the inner friction-reducing textured or structured surface is provided over the entire length of the working channel tube. The inner friction-reducing textured or structured surface may be uniform over the entire length of the working channel tube.

Alternatively, there may be a gradient or a stepped course of the friction-reducing textured or structured surface over the entire length. Further alternatively, the friction-reducing textured or structured surface may be locally more defined at special sections or may be only provided at special section. For example, it may be especially preferred to provide the friction-reducing textured or structured surface at a portion of the working channel tube which is arranged in the bending section of the insertion cord or at a portion of the working channel tube which is arranged directly adjacent the working channel access port/ the Y-connector.

It is to be understood, that the "entire length" of the working channel tube includes the whole working channel tube from the working channel access port to the distal tip unit.

Preferably, the inner diameter of the working channel tube is greater than 2 mm and smaller than 4.5 mm. Kinking is a problem that may depend on the inner diameter of the working channel tube, and it has been found that tubes having an inner diameter of 2 mm or more may not have the problem of kinking.

Preferably, the working channel tube is a seamless working channel tube.

Preferably, the endoscope of the present disclosure may be a single use endoscope. Roughening the surface of the working channel tube of conventional reusable endoscopes would make cleaning and sterilization of the endoscope considerably more difficult or even impossible. It is therefore advantageous to design the endoscope as a disposable item.

In another aspect, the inner friction-reducing textured or structured surface may be obtained by:
- providing an elongated hollow tube body, preferably via extrusion, having an inner surface;
- inserting a mandrel having a texture or structure on its outer surface into the elongated hollow tube body; and
- pressing the elongated hollow tube body against the mandrel with a force sufficient for transferring the texture or structure of the mandrel onto the inner surface of the elongated hollow tube body.

In other words the inner friction-reducing textured or structured surface may be obtained in essentially three steps:
In a first step, the elongated hollow tube body, which consists of at least one layer, optionally of several layers, may be provided. The elongated hollow tube body is to be understood as a blank which is essentially unprocessed after an initial forming step. Preferably, the elongated hollow tube body may already have a desired length of the subsequent working channel tube. The elongated hollow tube body is preferably made of at least one thermoplastic by means of extrusion and preferably has a circular cylindrical shape. Preferably, the initial forming step is the extrusion step.

In a second step, the mandrel may be inserted into the elongated hollow tube body. The diameter of the mandrel essentially corresponds to an inner diameter of the elongated hollow tube body. The diameter of the mandrel may preferably be a maximum of 20% smaller than the inner diameter of the elongated hollow tube body. An outer surface of the mandrel which may also be designated as circumferential or lateral or mantle or shell surface of the mandrel may be formed with a texturing or structuring. The texturing or structuring of the outer surface of the mandrel corresponds to a negative form of the texturing or structuring of the inner surface of the working channel tube to be generated, i.e. formed. The texturing or structuring of the outer surface of the mandrel may be a roughened surface with an irregular pattern or may comprise a regular pattern such as a rhombic pattern, a diamond pattern, a pyramidal pattern, etc. The texturing or structuring of the mandrel may be uniform over the entire length of the mandrel. Alternatively, the texturing or structuring of the mandrel may differ or change in sections. Further alternatively, the texturing or structuring of the mandrel may differ or change continuously.

The mandrel may be made of metal, preferably steel, particularly preferably stainless steel.

The mandrel may at least be the same length as the elongated hollow tube body, preferably the mandrel may be longer than the elongated hollow tube body.

In a third step, the elongated tube body may be pressed against the mandrel. Pressing may be performed with a force sufficient to imprint the texturing or patterning of the mantle surface of the mandrel into the inner surface of the elongated hollow tube body. Pressing is preferably carried out with a press body. The press body and the mandrel may be moved relative to each other. A vector of the pressing force may be oriented normal to a central axis of the mandrel. The pressing force may be applied radially all around the mandrel at the same time or applied sequentially. Furthermore, the pressing force may be applied over the full length of the elongated hollow tube body at the same time, section by section or traversing. The pressing force may be applied linearly increasing, increasing in steps or be describable by a mathematical formula.

The pressing force may be applied mechanically, pneumatically, hydraulically or in any other suitable way. The pressing force may be such that an overpressing takes place. This means that the pressing force causes a greater indentation of the inner surface of the working channel tube than desired, so that a restoring force or a springback of the material of the working channel tube may be taken into account.

Preferably, the elongated hollow tube body may be pressed against the mandrel by one or more rollers or wheels moving along an axial direction of the elongated hollow tube body, potentially at different positions around the circumference of the tube.

In other words, the press body described in step three may be one or more rollers or wheels, which apply the constant pressing force to the elongated hollow tube body and travel/move parallel to the mandrel over the full length of the elongated hollow tube body.

Further, the object of the present disclosure is solved by a system comprising an endoscope according to any of the above aspects; and a display device, preferably in the form of a monitor.

Furthermore, the object of the present disclosure is solved by a method for producing a working channel tube of an endoscope, in particular of an endoscope according to any of the above aspects, the method comprising the steps:
- providing an elongated hollow tube body, preferably via extrusion, having an inner surface;
- inserting a mandrel having a texture or structure on its outer surface into the elongated hollow tube body; and
- pressing the elongated hollow tube body against the mandrel with a force sufficient for transferring the texture or structure of the mandrel onto the inner surface of the elongated hollow tube body, in order to obtain an inner friction-reducing textured or structured surface of the working channel tube.

In a first step, the elongated hollow tube body, which consists of at least one layer, optionally of several layers, is provided. The elongated hollow tube body is to be understood as a blank which is essentially unprocessed after an initial forming step. Preferably, the elongated hollow tube body may already have a desired length of the subsequently formed working channel tube. The elongated hollow tube body is preferably made of at least one thermoplastic by means of extrusion and preferably has a circular cylindrical shape. Preferably, the initial forming step is the extrusion step.

In a second step, the mandrel is inserted in the elongated hollow tube body. The diameter of the mandrel essentially corresponds to an inner diameter of the elongated hollow tube body. The diameter of the mandrel may preferably be a maximum of 20% smaller than the inner diameter of the elongated hollow tube body. An outer surface of the mandrel which may also be designated as circumferential or lateral or mantle or shell surface of the mandrel is formed with a texturing or structuring. The texturing or structuring of the lateral surface of the mandrel corresponds to a negative form of the texturing or structuring of the inner surface of the working channel tube to be generated, i.e. formed. The texturing or structuring of the lateral surface of the mandrel may be a roughened surface with an irregular pattern or may comprise a regular pattern such as a rhombic pattern, a diamond pattern, a pyramidal pattern, etc. The texturing or structuring of the mandrel may be uniform over the entire length of the mandrel. Alternatively, the texturing or structuring of the mandrel may differ or change in sections. Further alternatively, the texturing or structuring of the mandrel may differ or change continuously.

The mandrel may be made of metal, preferably steel, particularly preferably stainless steel.

The mandrel may at least be the same length as the elongated hollow tube body, preferably the mandrel may be longer than the elongated hollow tube body.

In a third step, the elongated tube body is pressed against the mandrel. Pressing is performed with a force sufficient to imprint the texturing or patterning of the outer surface of the mandrel into the inner surface of the elongated hollow tube body. Pressing is preferably carried out with a press body. The press body and the mandrel may be moved relative to each other. A vector of the pressing force may be oriented normal to a central axis of the mandrel. The pressing force may be applied radially all around the mandrel at the same time or applied sequentially. Furthermore, the pressing force may be applied over the full length of the elongated hollow tube body at the same time, section by section or traversing. The pressing force may be applied linearly increasing, increasing in steps or be describable by a mathematical formula.

The pressing force may be applied mechanically, pneumatically, hydraulically or in any other suitable way. The pressing force may be such that an overpressing takes place. This means that the pressing force causes a greater indentation of the inner surface of the working channel tube than desired, so that a restoring force or a springback of the material of the working channel tube may be taken into account.

Preferably, the method may further comprise a fourth step of pressing the elongated hollow tube body against the mandrel by one or more rollers or wheels moving along an axial direction of the elongated hollow tube body, potentially at different positions around the circumference of the tube.

In other words, the press body described in step three may be one or more rollers or wheels, which apply the constant pressing force to the elongated hollow tube body and, in the fourth step travel/move parallel to the mandrel over the full length of the elongated hollow tube body.

Preferably, the method may further comprise a fifth step of rotating the elongated hollow tube body and/or the mandrel relative to the press body, in particular the one or more rollers or wheels. By rotating the mandrel, another circumferential section of the elongated hollow tube body is brought into contact with the press body, in particular the one or more rollers or wheels.

Alternatively, the at least one roller or wheel may be rotated relative to the mandrel. Further alternative embodiments are conceivable in which a number of rollers or wheels are arranged around the mandrel in such a way that the entire inner surface of the working channel tube is textured or structured when they are moved along once.

Preferably, the fourth and fifth steps are performed alternately until the entire inner surface of the elongated hollow tube body is textured or patterned and the elongated hollow tube body has become the working channel tube.

In other words, the elongated hollow tube body may be rotated around its center axis and the one or more rollers may be moved along the axial direction of the elongated hollow tube body more than once.

Preferably, the method may further comprise a step of heating up the elongated hollow tube body before or during pressing the elongated hollow tube body against the mandrel. The heating may be performed by a heatable mandrel. Alternatively or additionally, heaters, in particular radiant heaters, may be provided circumferentially of the elongated hollow tube body or adjacent to the rollers or wheels. Further alternatively or additionally, the rollers or wheels may be heated. Furthermore, in addition or alternatively, the elongated hollow tube body may already be heated before being positioned on the mandrel.

Preferably, the rollers or wheels may be rotatably mounted on a sled, i.e. carriage and the sled may be configured to travel parallel to the mandrel.

Preferably, the at least one roller or wheel moves at a constant speed along the mandrel.

Preferably, the one or more rollers or wheels may comprise a grooved outer circumference, the grooved outer circumference having a radius adapted to a radius of the mandrel.

In other words the one or more rollers or wheels may comprises a groove extending radially inward on the circumferential surface. The groove may have a substantially arcuate cross-sectional shape. The geometry of the groove may be adapted to the geometry of the mandrel in such a way that a radius of the groove corresponds to the radius of the mandrel to a deviation of less than 10%. Alternatively or additionally, the geometry of the groove may be adapted to a geometry of the elongated hollow tube body. The geometry of the groove may be adapted to the geometry of the elongated hollow tube body in such a way that a radius of the groove corresponds to the radius of the elongated hollow tube body to a deviation of less than 10%.

Preferably, the rollers or wheels may have a structured or textured surface on their peripheral surface. Specifically, the surface of the groove may be structured or textured. When a roller or wheel with such a structured surface travels along the elongated hollow tube body, the outer surface of the elongated hollow tube body may be structured or textured. In this way, the inner surface and the outer surface may be structured, textured or roughened simultaneously. The degree of structuring, texturing or roughening of the inner surface and the outer surface may differ or may correspond to each other.

### Brief description of figures

The disclosure is explained in more detail below using preferred embodiments and referring to the accompanying figures.
Fig. 1 shows a schematic view of an endoscope according to the present disclosure.
Fig. 2 shows a schematic view of a working channel tube of the endoscope according to a first embodiment of the disclosure.
Fig. 3 shows a schematic view of the working channel tube of the endoscope according to a second embodiment of the disclosure.
Fig. 4 shows a longitudinal sectional view of the working channel tube of the endoscope in a single layer embodiment.
Fig. 5 shows a longitudinal sectional view of the working channel tube of the endoscope in a double layer embodiment.
Fig. 6 shows a longitudinal sectional view of the working channel tube of the endoscope in a triple layer embodiment.
Fig. 7 shows a perspective view of a roller for forming an inner surface of the working channel tube.
Fig. 8 shows a front view of the roller for forming the inner surface of the working channel tube.
Fig. 9 shows a schematic view of a device for forming the inner surface of the working channel tube.
Fig. 10 shows a schematic view of a mandrel.
Fig. 11 shows a sectional view of two rollers and the mandrel forming the inner surface of the working channel tube.
Fig. 12 shows a perspective view of the roller interacting with the working channel tube.

### Detailed description of preferred embodiments

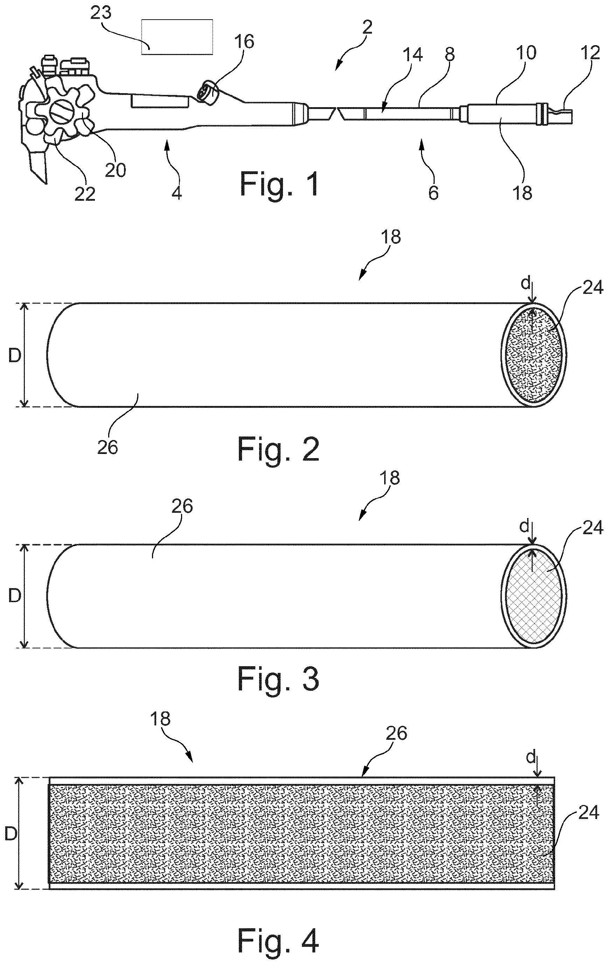

Fig. 1 shows a schematic view of an endoscope 2, which is preferably a single-use endoscope. The endoscope 2 has a proximal endoscope handle 4 and an insertion cord 6 extending distally from the endoscope handle 4. The insertion cord 6 is configured to be inserted into a patient's body cavity and comprises an insertion tube 8, an actively bendable bending section 10 and a distal tip unit 12. Embodiments of the endoscope 2 are also conceivable which are formed without bending section 10. The endoscope 2 further comprises a working channel 14 which extends from a working channel access port 16 provided at the endoscope handle 4 to the distal tip unit 12. The working channel 14 comprises a working channel tube 18, which is arranged inside the endoscope handle 4, the insertion tube 8 and the bending section 10. The endoscope handle 4 comprises two operating units 20, 22 formed as wheels for steering the bending section 10 of the insertion cord 6. In particular, a rotation/ turning force may be applied to both the first operating unit 20 and the second operating unit 22 by a user in order to bend the bending section 10 in two bending planes/ four directions. The endoscope 2 may alternatively be formed as a one-plane bending endoscope which may be bent only in two, preferably opposite, directions and which has only one operating unit 20, 22. A display unit 23 may be connected to the endoscope 2.

The working channel tube 18 provided inside the endoscope handle 4, the insertion tube 8 and the bending section 10 is a specific working channel tube 18 according to the present disclosure and comprises an inner friction-reducing textured or structured surface 24 (see e.g. Fig. 2). It is to be understood that it is also conceivable that only parts of the working channel tube 18 comprise the inner friction-reducing textured or structured surface 24. For example, the working channel tube 18 may have the inner friction-reducing textured or structured surface 24 in an area of the bending section 10 or in an area of the working channel access port 16.

It should be noted that all figures of the working channel tube 18 are to be understood schematically and the size and dimensional relationships shown are not to be understood as a limitation. For example, a diameter D of the working channel tube 18 compared to a length of the working channel tube 18 is shown much larger in the figures in order to better represent properties of the working channel tube 18.

The working channel tubes 18 disclosed herein are seamless and manufactured by extrusion. However, other manufacturing processes are also conceivable. It is also conceivable to form the working channel tube 18 with a seam.

Fig. 2 shows a schematic view of the working channel tube 18 according to a first embodiment. The working channel tube 18 has an essentially tubular geometry with an inside surface 24 and an outside surface 26. A wall thickness d of the working channel tube 18 is small compared to the diameter D of the working channel tube 18. The working channel tube 18 is preferably made of a thermoplastic material. The inside surface 24 of the working channel tube 18 comprises a roughened structure in order to reduce friction between medical tools and the like passing through the working channel tube 18.

"Roughness" refers to an unevenness of the surface height. In other words, the inside surface 24 may have (irregular) protrusions and/or indentations that reduce a contact area and thus the friction between the medical tools and the inside surface 24 of the working channel tube 18. An example of such a surface is a lotus effect surface. In the first embodiment disclosed herein, the inside surface 24 is rougher than the outside surface 26. However, it is also conceivable that the outside surface 26 is also roughened.

Fig. 3 shows a schematic view of the working channel tube 18 according to a second embodiment. The working channel tube 18 according to the second embodiment corresponds in its (macro-) geometry to the working channel tube 18 in Fig. 2. However, the working channel tube 18 according to the second embodiment comprises a regularly structured surface on its inside surface 24. In other words, the inside surface 24 of the second embodiment is a regularly patterned surface. In the second embodiment disclosed herein, the inside surface 24 is a uniform, geometrically regular structured inside surface 24. Specifically, the inside surface 24 is formed with a rhombic pattern. Other possible patterns are a diamond pattern, a pyramid pattern and the like. In the second embodiment disclosed herein, the inside surface 24 is more structured/ textured than the outside surface 26. However, it is also conceivable that the outside surface 26 is also structured/ textured.

Furthermore, it is conceivable that the working channel tube 18 according to the second embodiment has a regularly structured outside surface 26. It is also conceivable that the working channel tube 18 according to the second embodiment has a roughened outside surface 26.

Fig. 4 shows a longitudinal sectional view of the working channel tube 18 according to the first embodiment in a single layer embodiment. In other words, the outside surface 26 is formed on the same layer as the inside surface 24. In yet other words, the working channel tube 18 is formed as a single piece of material. The wall thickness d and the diameter D are constant along a length of the working channel tube 18. A roughness parameter of the inside surface 24, e.g. Ra or Rz, which describes a degree of roughness, is constant over the length of the working channel tube in the first embodiment disclosed herein.

However, embodiments are also conceivable in which the roughness parameter of the inside surface 24 changes stepwise or linearly or in a combination of stepwise and linearly over the length of the working channel tube 18.

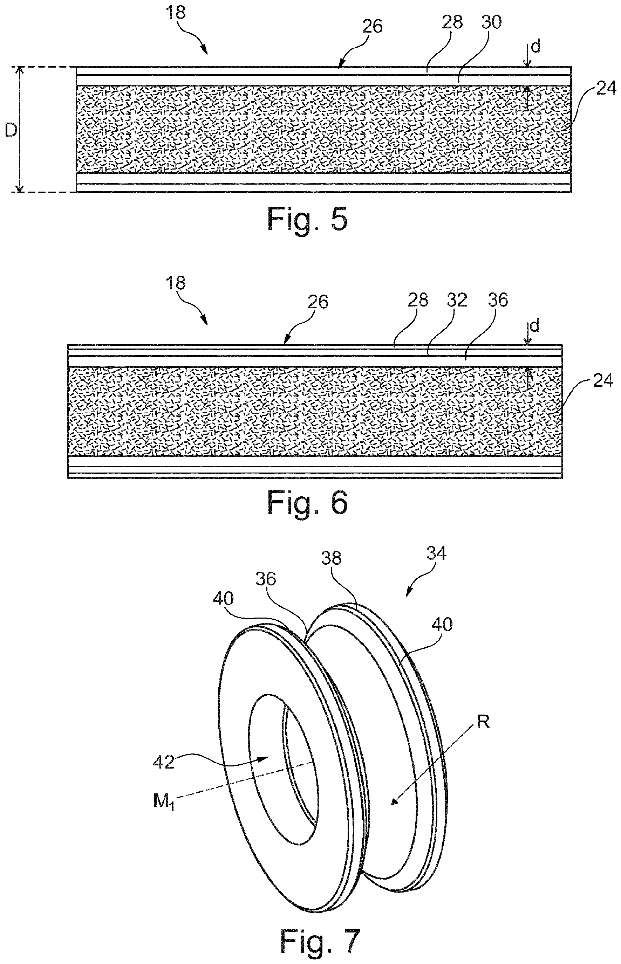

Fig. 5 shows a longitudinal sectional view of the working channel tube 18 according to the first embodiment in a double layer embodiment. Only differences to the single-layer design are discussed in the following. The outside surface 26 is formed on a different layer as the inside surface 24. Specifically, the outside surface 26 is formed on an outside layer 28 and the inside surface 24 is formed on an inside layer 30. The outside layer 28 and the inside layer 30 are connected to each other in a force-fit manner or adhesively. The outside layer 28 and the inside layer 30 may differ in a layer thickness and material.

Fig. 6 shows a longitudinal sectional view of the working channel tube 18 according to the first embodiment in a triple layer embodiment. Only differences to the single-layer design and to the double layer design are discussed in the following. A bridge layer 32 is formed between the outside layer 28 and the inside layer 30. The bridge layer 32 may differ from the outside layer 28 and from the inside layer 30 in a layer thickness and material. The outside layer 28, the inside layer 30 and the bridge layer 32 are connected to each other in a force-fit manner or adhesively. The bridge layer 32 may be configured as an interconnecting layer.

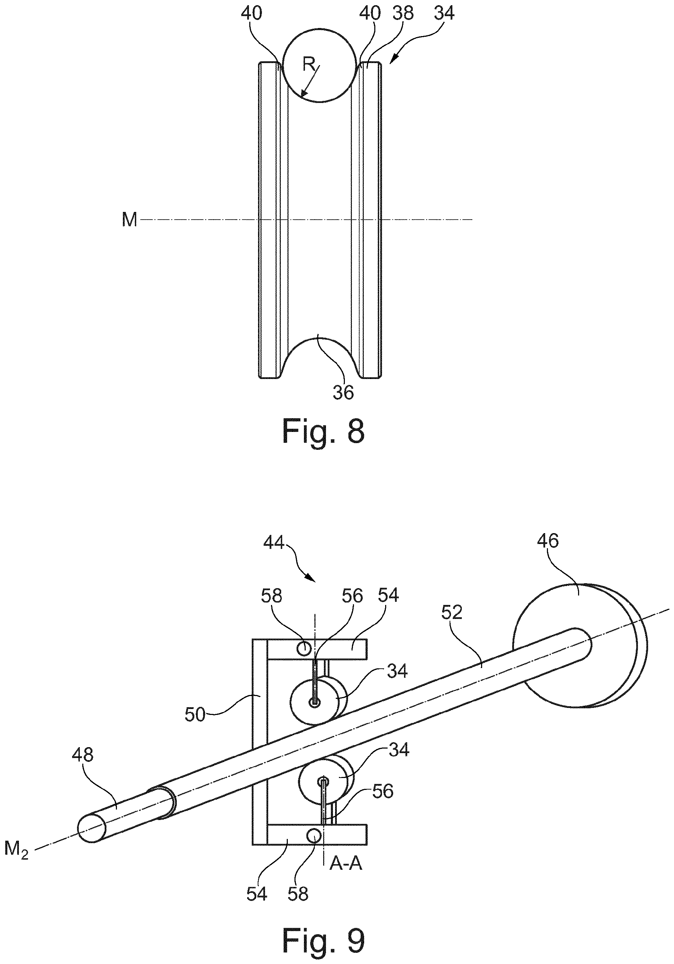

Fig. 7 and Fig. 8 show a roller 34, which is used to form the inside surface 24 of the working channel tube 18. The forming process will be described later with reference to Figures 9 to 12. The roller 34 has a wheel-shaped geometry with a groove 36 formed in the peripheral surface 38 of the roller 34. The groove 36 has a substantially arcuate geometry with a radius R. The radius R of the groove 36 is matched/ adapted to the geometry/curvature of the working channel tube 18 and/or to a mandrel (48) described later. Specifically, the radius R of the groove 36 may be substantially half the diameter D of the working channel tube 18. The radius R of the groove 36 is preferably half the diameter D of the working channel tube 18 +/- 10%. A chamfer 40 is formed at a transition from the circumferential surface 38 to the groove 36. The roller 34 is configured to rotate about a center axis M1. A circular-cylindrical axle mount 42 is formed on the center axis M1. The roller 34 is made of metal, in particular (stainless) steel. Alternatively, the roller 34 may also be made of ceramic.

Fig. 9 shows a schematic view of a device 44 for forming the inner surface 24 of the working channel tube 18. Regarding the device 44, it should be repetitively noted that the dimensions and size ratios shown here are purely for illustration purposes and deviate from the actual dimensions and size ratios.

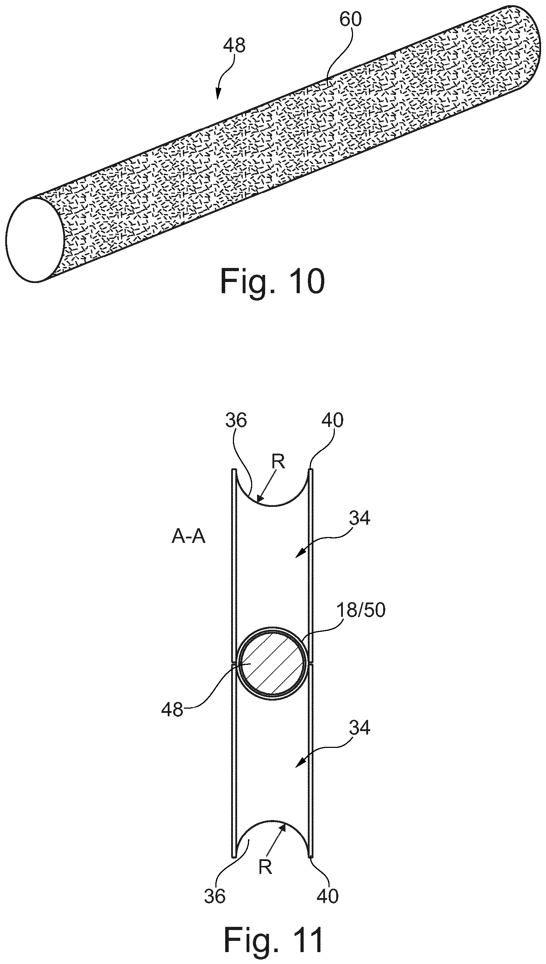

The device 44 comprises a clamp 46 provided rotatable about a central axis M2, a mandrel 48 formed on the central axis M2 and extending along the central axis M2 and non-rotatably connected to the clamp 46, and a sled 50 formed with two rollers 34 and movable parallel to the central axis M2. The clamp 46 is provided to clamp a tubular working channel tube blank 52 to the clamp 46 in a non-rotatably manner. The working channel tube blank 52 is mounted onto the mandrel 48.The mandrel 48 will be described in Fig. 10 in more detail. The sled 50 has substantially a U-shape that embraces the mandrel 48. Roller supports 56 are formed on opposite arms 54 of the sled 50. The roller supports 56 face each other and a roller 34 is formed at the end of each roller support 56 remote from the respective arm 54 of the sled 50. Each of the arms 54 is formed with a force generating device 58. Alternatively, the force generating device 58 may also be formed at another position on the sled 50. In the embodiment disclosed herein, the sled 50 is designed with two rollers 34. However, embodiments with only one roller 34 or with three or more rollers 34 are also conceivable. The rollers 34 in the embodiment disclosed herein are formed directly opposite each other on the roller support 56. However, embodiments are also conceivable in which the rollers 34 are mounted offset from one another.

Fig. 10 shows a schematic view of the mandrel 48. The mandrel 48 has a circular-cylindrical geometry with an outer (shell) surface 60. The outer surface 60 has a surface structure which is configured to shape the inner surface 24 of the working channel tube 18. In other words, the outer surface 60 of the mandrel 48 is provided with a rough surface with protrusions and/ or recesses. In the embodiment disclosed herein, the mandrel 48 is formed with a rough outer surface 60 which forms/ shapes an inside surface 24 of the working channel tube 18 according to the first embodiment. In order to form an inner surface 24 in accordance with the second embodiment of the working channel tube 18, a mandrel 48 is provided having a corresponding structure or pattern or the like formed on its outer surface 60. The mandrel 48 is preferably made of metal, particularly preferably stainless steel.

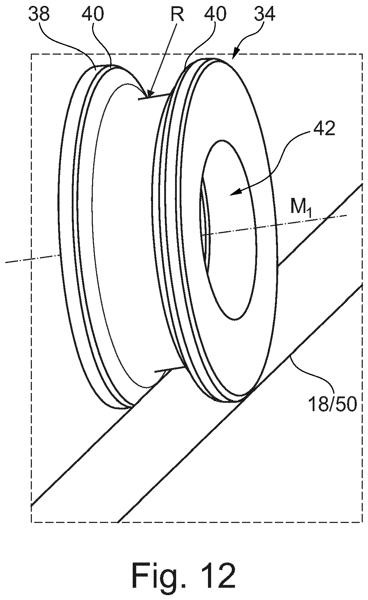

Fig. 11 shows a cross-section A-A indicated in Fig. 9. Fig. 12 shows an embodiment of a single roller 34 in engagement with the working channel tube blank 52.

In the following, the forming of the inner surface 24 of the working channel tube 18 with the device 44 is described with reference to Fig. 9 to Fig. 12. Since it is not possible to make a clear distinction between the working channel tube 18 and the working channel tube blank 50 during the process, the working channel tube 18 will always be referred to in the following as soon as a first machining/forming/process step has been performed with the working channel tube blank 50. In general, the working channel tube blank 50 is the tube-shaped body in which no machining of the inside surface 24 has yet taken place, and the working channel tube 18 is the tube-shaped body in which the inside surface 24 has been machined /formed.

In a first step, the working channel tube blank 52, which was produced preferably by extrusion, is placed on the mandrel 48 and clamped by the clamp 46 so as to be non-rotatably with the clamp 46 and the mandrel 48. A length of the mandrel 48 is larger than a length of the working channel tube 18.

Then, in a second step, the rollers 34 are brought into contact with the outside surface 26 of the working channel tube 18. Specifically, the rollers 34 are brought into contact with the outside surface 26 of the working channel tube 18 by moving the roller supports 56 in such a way, that the groove 36 of the respective roller 34 partially surrounds the outside surface 26 of the working channel tube 18 (see Fig. 11 and Fig. 12).

In a third step, the rollers 34 are pressed against the working channel tube 18 with a force sufficient to transfer a surface topography of the mandrel 48 to the inner surface 24 of the working channel tube 18.

In a fourth step, the sled with the rollers 34 is moved along/parallel to the working channel tube 18, while the force is still applied to the rollers 34 and the rollers 34 are still engaged with the working channel tube 18. The sled 50 is moved in such a way, that the rollers 34 move over the full length of the working channel tube 18. In this process, the surface topography of the mandrel 48 is pressed/formed into the inside surface 24 of the working channel tube 18 in a longitudinal strip section of the inside surface 24.

In a fifth step, the clamp 46 with the working channel tube 18 and the mandrel 48 is rotated by a defined angle around the central axis M2. The rotation is implemented by a drive (not shown) connected to the clamp 46 or formed in the clamp 46. Then the fourth step is repeated.

The defined angle of rotation is set in such a way that one longitudinal strip is formed directly without spacing or even overlapping next to the next one. The fourth step and the fifth step are repeated at least until the entire inside surface 24 of the working channel tube 18 has been treated/formed.

The working channel tube 18 may additionally be heated during the process. For example, the mandrel 48 may be heated or the working channel tube 18 may be heated from the outside, for example by radiant heaters or the like formed on the sled 50.

### Reference signs

- 2: endoscope
- 4: endoscope handle
- 6: insertion cord
- 8: insertion tube
- 10: bending section
- 12: distal tip unit
- 14: working channel
- 16: working channel access port
- 18: working channel tube
- 20: operating unit
- 22: operating unit
- 23: display unit
- 24: inner surface
- 26: outside surface
- 28: outside layer
- 30: inside layer
- 32: bridge layer
- 34: roller
- 36: groove
- 38: peripheral surface
- 40: chamfer
- 42: axle mount
- 44: device
- 46: clam p
- 48: mandrel
- 50: sled
- 52: working channel tube blank / elongated hollow tube body
- 54: arm
- 56: roller support
- 58: force generating device
- 60: outer surface
- D: diameter
- R: radius
- M1: center axis
- M2: central axis

## Claims

1. An endoscope (2) comprising:
a proximal endoscope handle (4) or interface comprising a handle or interface housing and a working channel access port (16);
an insertion cord (6) extending distally from the endoscope handle (4) or interface, configured to be inserted into a patient's body cavity and comprising at least an insertion tube (8) and a distal tip unit (12); and
a working channel (14) extending from the working channel access port (16) of the endoscope handle (4) or interface to the distal tip unit (12) of the insertion cord (6) and comprising a working channel tube (18) arranged at least in sections inside the insertion cord (6), the working channel tube (18) comprising an inner friction-reducing textured or structured surface (24) and an outer surface (26).

2. The endoscope (2) according to claim 1, wherein the inner friction-reducing textured or structured surface (24) is a roughened surface.

3. The endoscope (2) according to claim 1 or 2, wherein the inner friction-reducing textured or structured surface (24) is rougher than the outer surface (26).

4. The endoscope (2) according to any one of claims 1 to 3, wherein the working channel tube (18) comprises an inner layer (30) or tube having the inner friction-reducing textured or structured surface (24) and an outer layer (28) or tube having the outer surface (26).

5. The endoscope (2) according to any one of claims 1 to 4, wherein the inner friction-reducing textured or structured surface (24) has an average roughness value Ra, and the average roughness value Ra is greater than or equal to 1.0 µm, preferably greater than or equal to 1.5 µm.

6. The endoscope (2) according to any one of claims 1 to 5, wherein the inner friction-reducing textured or structured surface (24) is provided over an entire length of the working channel tube (18).

7. The endoscope (2) according to any one of claims 1 to 6, wherein the inner friction-reducing textured or structured surface (24) is obtained by:
providing an elongated hollow tube body (52), preferably via extrusion, having an inner surface;
inserting a mandrel (48) having a texture or structure on its outer surface (60) into the elongated hollow tube body (52); and
pressing the elongated hollow tube body (52) against the mandrel (48) with a force sufficient for transferring the texture or structure of the mandrel onto the inner surface of the elongated hollow tube body (52).

8. The endoscope (2) according to claim 7, wherein the elongated hollow tube body (52) is pressed against the mandrel (48) by one or more rollers (34) or wheels moving along an axial direction of the elongated hollow tube body (52).

9. A system comprising: an endoscope (2) according to any one of claims 1 to 8; and a display unit (23).

10. A method for producing a working channel tube (18) of an endoscope (2), in particular of an endoscope (2) according to any one of claims 1 to 8, the method comprising the steps:
providing an elongated hollow tube body (52), preferably via extrusion, having an inner surface;
inserting a mandrel (48) having a texture or structure on its outer surface (60) into the elongated hollow tube body (52); and
pressing the elongated hollow tube body (52) against the mandrel (48) with a force sufficient for transferring the texture or structure of the mandrel (48) onto the inner surface of the elongated hollow tube body (52), in order to obtain an inner friction-reducing textured or structured surface (24) of the working channel tube (18).

11. The method according to claim 10, further comprising the step:
pressing the elongated hollow tube body (52) against the mandrel (48) by one or more rollers (34) or wheels moving along an axial direction of the elongated hollow tube body (52).

12. The method according to claim 11, further comprising the steps:
rotating the elongated hollow tube body (52) around its center axis (M2); and
moving the one or more rollers (34) or wheels more than once along the axial direction of the elongated hollow tube body (52).

13. The method according to any one of claims 10 to 12, further comprising the step:
heating up the elongated hollow tube body (52) before or during pressing the elongated hollow tube body (52) against the mandrel (48).

14. The method according to any one of claims 10 to 13, wherein a length of the mandrel (48) is equal to or greater than a length of the elongated hollow tube body (52).

15. The method according to any one of claims 10 to 14, wherein the one or more rollers (34) or wheels comprise a grooved outer circumference (38), the grooved outer circumference (38) having a radius (R) adapted to a radius or diameter (D) of the mandrel (48).
